# EUROPEAN PATENT APPLICATION

(11) **EP 0 623 358 A2**
(43) Date of publication of application: **09.11.1994**
(21) Application number: 94301948.9
(22) Date of filing: 18.03.1994
(51) Int. Cl.: A61M 5/32

(54) **Needle sheaths**

(30) Priority: 16.04.1993 US 47182
(71) Applicant: SMITHS INDUSTRIES MEDICAL SYSTEMS INC., Keene, New Hampshire 03431-0724 (US)
(72) Inventor: Hollister, William Hermann, East Sullivan, New Hampshire 03445 (US)
(74) Representative: Flint, Jonathan McNeill

(57) **Abstract**

A safety device for a needle 16 has a housing 6 hinged to a base 2 attached to the needle. The housing 6 has a lock 22, for engaging the needle 16, and a fluid-absorbing material such as a foam 26, which contacts the needle before engagement of the lock. The needle 16 can pass through the fluid-absorbing material 26, which absorbs any fluid collected at the tip of the needle.

## Description

This invention relates to safety devices for use with a needle.

The invention is more particularly concerned with sheaths for preventing the tip of a hypodermic needle being exposed after use.

In US Patent 4,982,842 there is disclosed a safety needle device comprising a housing hinged to a base of the device. The housing is pivotable to a position in alignment with a needle mated to the device so as to enclose the needle. This device has proven to be quite effective in preventing accidental needle pricks.

Fluid, such as blood or medicament can collect inside or outside the needle after use and it is possible for this fluid to be splattered, splashed or aerosolized into the atmosphere when the protective housing is pivoted to enclose the needle. This has been found to be particularly the case when the housing has an integrated lock, such as a hook, that snaps onto the needle as the housing is pivoted over the needle. The fluid that is aerosolized or atomized may be contaminated, such as, for example, contaminated blood carrying the HIV virus.

It is an object of the present invention to provide a needle sheath with a reduced risk of splattering.

According to one aspect of the present invention there is provided a safety device of the above-specified kind, in that the device includes a base secured with the needle, a housing hingedly attached to the base that can be pivoted to a position in substantially alignment along the longitudinal axis of the needle for substantially sheathing the needle, and an absorbing material on the housing arranged to absorb fluid collected at the needle as the housing is pivoted to the alignment position and the needle contacts the absorbing material.

The device preferably includes a lock integral with the housing, the lock fixedly retaining the needle within the housing once the housing is pivoted to the alignment position. The absorbing material may be located on an end of the housing remote from the base, the absorbing material being configured to contact the needle as the housing is pivoted to the alignment position, before the needle contacts the lock. Alternatively, the device may include a member on the housing that cooperates with second member on the base and prevents relative movement between the housing and the base once the housing is pivoted to the alignment position. The absorbing material may be configured to contact the needle before the first and second members fully cooperate to prevent relative movement between the housing and the base. The absorbing material preferably comprises a fluid-absorbing material with sufficient yield to allow the needle to traverse therethrough when the housing is pivoted to the alignment position. The absorbing material may be a foam, cotton or a sponge.

Examples of needle sheath assemblies according to the present invention will now be described, by way of example, with reference to the accompanying drawings, in which:
- Figure 1: is a partially cut-away side elevation view of the needle sheath assembly including a safety device with its sheath pivoted away from the needle;
- Figure 2: is a partially cut-away elevation view of the assembly Figure 1 with the needle sheath pivoted to a position in alignment along the longitudinal axis of the needle/base;
- Figure 3: is a plan view of the device with the sheath pivoted away from the needle;
- Figure 4: is a perspective view of the base and lower portion of the modified device using a different locking mechanism; and
- Figure 5: is a perspective view of the base and lower portion of the another modified device using another different locking mechanism.

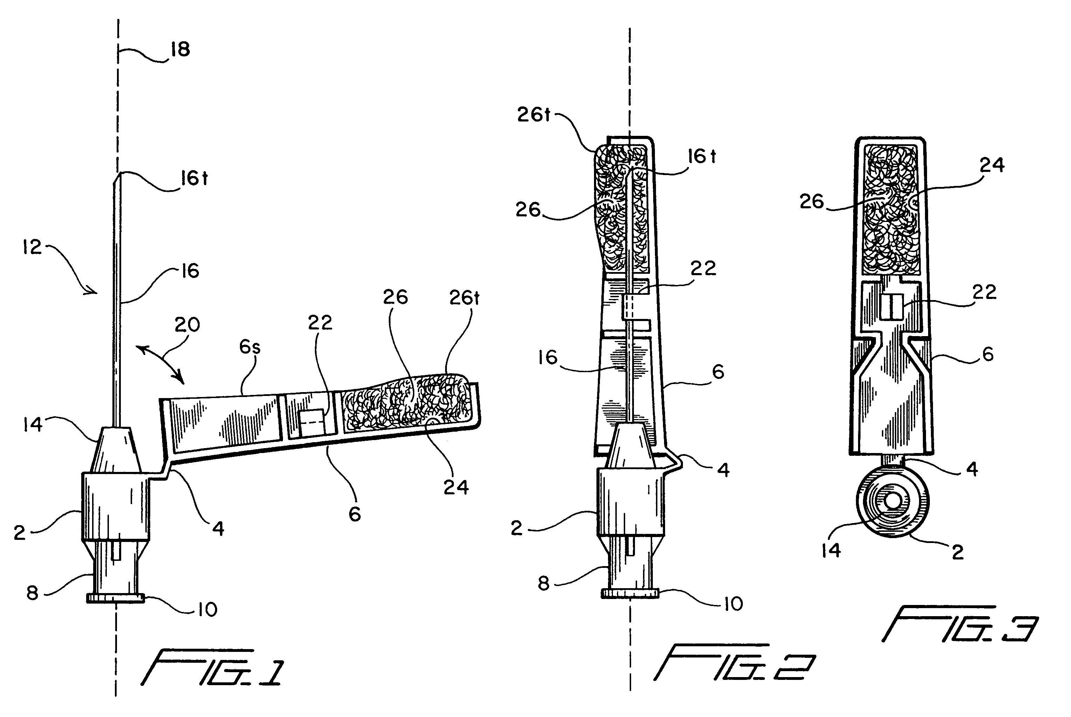

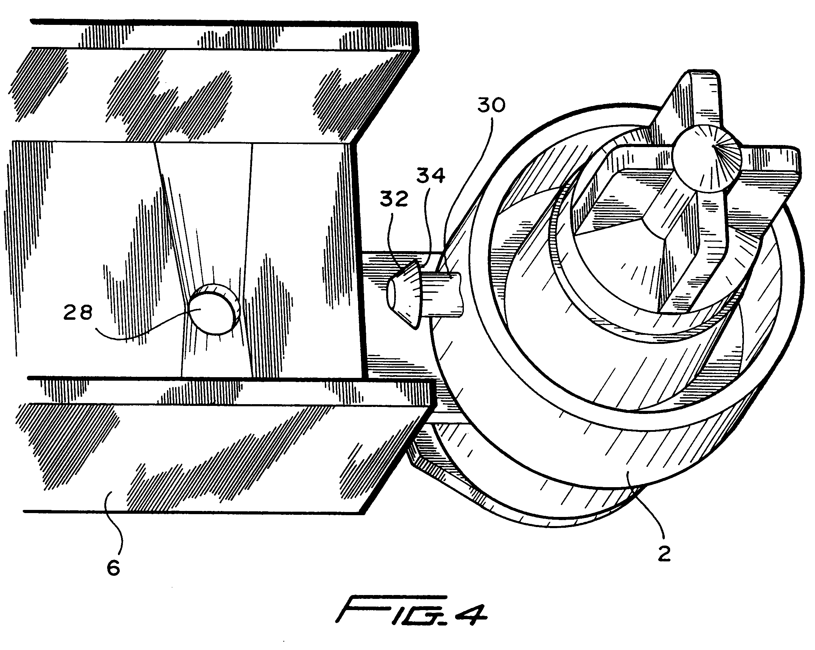

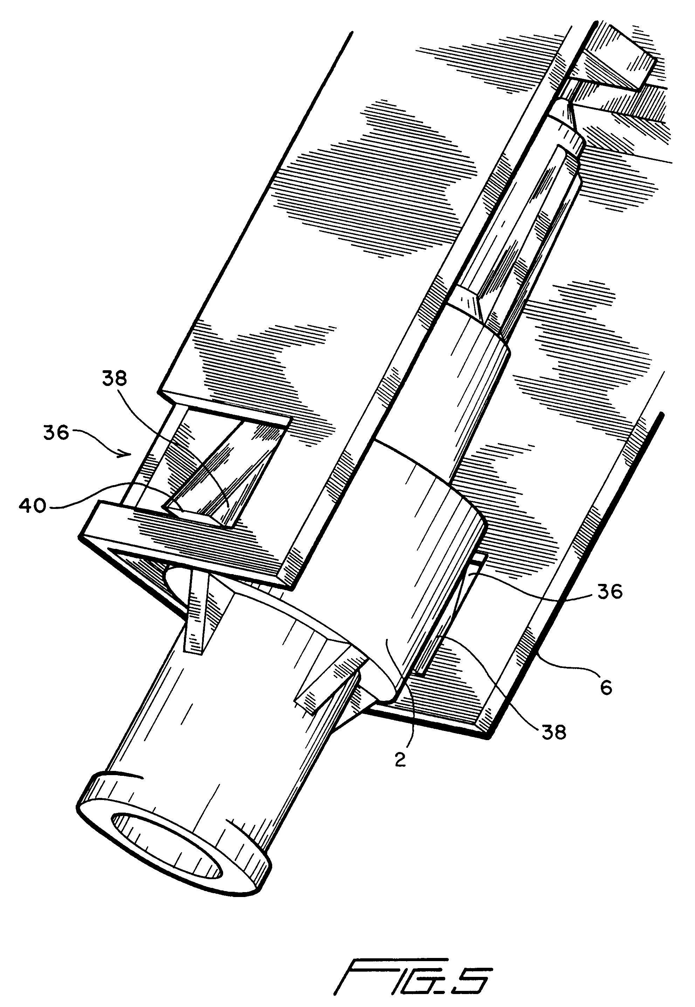

With reference to Figures 1 to 3, there is shown a needle sheath assembly including a safety device having a base 2 connected by means of a flexible strap or living hinge 4 to a housing or sheath 6. The base 2 has an extension 8 with a circumferential lip 10 at its lower end for mating with a fluid container, for example a syringe (not shown). The needle sheath assembly includes a needle assembly 12 having a needle 16 and is mated to the other end of the base 2, by way of a hub 14. Thus, the needle 16 is fixed to the base 2 along a longitudinal axis 18.

The sheath 6 is shown partially cut away and is connected to the base 2 by the hinge 4, such that it is pivotable bi-directionally as indicated by bi-directional arrow 20. In Figure 1, the sheath 6 is shown to be at a position away from the longitudinal axis 18 so that the needle 16 may be used either to supply medicament to or withdraw blood from a patient. After use, the needle 16 may have a droplet of blood at or close to its tip 16t.

If a conventional needle sheath were used, there would be a risk that the droplet of contaminated blood would be splashed or flicked into the atmosphere, especially if too vigorous a snapping motion were used. This could result in the blood droplet being aerosolized or atomized so that it forms a fine mist. Thus, an operator, or nearby bystanders, could be sprayed by the mist or droplet and potentially be exposed to contagious diseases possibly carried by the contaminated blood.

In the present invention, the sheath 6 has a locking mechanism, in the form of hook 22, integrated with the housing. For the sake of simplicity, only one hook 22 is shown although a plurality of hooks may also be used. The sheath 6 has a large channel 24 containing a fluid-absorbent material 26. The material 26 is fixed in the cavity provided by the channel 24, as for example by bonding. When the sheath 6 is pivoted toward the axis 18, the material 26 will be traversed by the needle 16 until the needle is retained by the hook 22, in the manner described in US 4,982,842. The material 26 may be comprised of any of the many fluid-absorbent materials such as foam, felt, cotton, paper, cloth, polyurethane foam or sponge. Other sufficiently soft fibrous materials that will yield to the relatively thin gauge flexible needle 16 when it is biased against them, could also be used. The material 26, furthermore, needs to be able to absorb at least its own weight in fluid.

In the present embodiment, the material 26 is configured to contact the needle tip 16t before the medial portion of needle 16 contacts the hook 22. Thus, whatever fluid collects at the needle tip 16t is absorbed by the material 26 before the needle 16 is retained by the hook 22, and fully enclosed by the sheath 6. In this way, there is no fluid to be flicked from the needle 16. As exaggeratedly shown in Figures 1 and 2, a part 26t of the material 26 extends away from the open surface 6s of the sheath 6.

In operation, when the sheath 6 is pivoted toward the axis 18 to envelop the needle 16, any contaminated droplets of blood collected at the tip 16t of the needle 16 first contact the material 26 at the part 26t and are absorbed thereby. Consequently, as the sheath 6 is further moved toward the axis 18 and is positioned in substantial alignment with the needle 16 and the base 2, there is no splattering or flicking of any contaminated fluid from the tip portion 16t. Figure 2 shows the retention of the needle 16 by the hook 22 and the enclosure of the upper portion of the needle 16 within the material 26.

Figures 4 and 5 show respective different embodiments in which relative movement between the sheath 6 and the base 2 is prevented once the sheath 6 has been pivoted to a position substantially in alignment with the axis 18. In the embodiments of Figures 4 and 5, no hook 22 is provided within the sheath 6. Accordingly, the needle 16 does not contact anything other than the material 26, even when the sheath 6 is fully rotated to its alignment position along the axis 18. The embodiments of Figures 4 and 5 each prevent blood droplets from dripping from the needle 16.

Specifically, in Figure 4, there is an opening 28 at the lower portion of the sheath 6 and an anchor 30 extending from the base 2. The opening 28 and the anchor 30 are situated such that when the sheath 6 is pivoted toward the base 2, the anchor 30, and particularly its graduated tip 32, passes through the opening 28. The tip 32 of the anchor 30 has a base 34 the diameter of which is greater than that of the opening 28. Consequently, once the sheath 6 has been positioned in its alignment position and the opening 28 traverses past the tip 32, the base 34 will prevent the sheath 6 from being pivoted in a direction away from the base 2. Accordingly, the sheath 6 remains at its alignment position enclosing needle 16, while the upper portion of the needle 16 (not shown in Figures 4 and 5) is buried within the material 26 and any fluid that may have been collected at needle tip 16t is absorbed by the material 26.

In the Figure 5 embodiment, instead of a single opening and a corresponding anchor, two openings 36 are provided at opposite sides of the lower portion of the sheath 6 and two corresponding tabs 38 are integrated at the base 2. Each of the tabs 38 has a slant configuration so that its base 40 prevents the sheath 6 from moving away from the base 2, once the sheath 6 has been pivoted in alignment with the needle 16. Thus, the openings 36 and tabs 38 cooperate to prevent further relative movement between the sheath 6 and the base 2 once the tabs 38 are snap-locked to the openings 36. The sheath 6 in the embodiment of Figures 4 and 5 could have material 26 configured in the same manner as that shown in Figures 1 and 2. Alternatively, material 26 could be configured not to extend beyond the surface 6s of the sheath 6 because there is no hook 22 integrated to the sheath 6 and because the needle 16 does not come into contact with anything other than the material 26. Thus, any fluid that may drip from tip 16t of needle 16 is absorbed by the material 26.

## Claims

1. A safety device for use with a needle, characterised in that the device includes a base (2) secured with the needle (16), a housing (6) hingedly attached to the base (2) that can be pivoted to a position in substantially alignment along the longitudinal axis of the needle (16) for substantially sheathing the needle, and an absorbing material (26) on the housing (6) arranged to absorb fluid collected at the needle (16) as the housing (6) is pivoted to the alignment position and the needle contacts the absorbing material (26).

2. A safety device according to Claim 1, characterised in that the device includes a lock (22) integral with the housing (6) and that the lock (22) fixedly retains the needle (16) within the housing (6) once the housing (6) is pivoted to the alignment position.

3. A safety device according to Claim 2, characterised in that the absorbing material (26) is located on an end of the housing (6) remote from the base (2), and that the absorbing material (26) is configured to contact the needle (16) as the housing (6) is pivoted to the alignment position, before the needle contacts the lock (22).

4. A safety device according to Claim 1, characterised in that the device includes a member (28, 36) on the housing (6) that cooperates with a second member (30, 32, 34) on the base (2) and prevents relative movement between the housing (6) and the base (2) once the housing is pivoted to the alignment position.

5. A safety device according to Claim 4, characterised in that the absorbing material (26) is configured to contact the needle (16) before the first and second members (28, 36 and 30, 32, 34, 38) fully cooperate to prevent relative movement between the housing (6) and the base (2).

6. A safety device according to any one of the preceding claims, characterised in that the absorbing material (26) comprises a fluid-absorbing material with sufficient yield to allow the needle (16) to traverse therethrough when the housing (6) is pivoted to the alignment position.

7. A safety device according to any one of the preceding claims, characterised in that the absorbing material comprises a foam.

8. A safety device according to any one of Claims 1 to 6, characterised in that the absorbing material comprises cotton or a sponge.
